Europäisches Patentamt

European Patent Office

Office européen des brevets

(11) Veröffentlichungsnummer: **0 226 789**
A2

## (12) EUROPÄISCHE PATENTANMELDUNG

(21) Anmeldenummer: 86115539.8

(22) Anmeldetag: 08.11.86

(51) Int. Cl.⁴: **A 41 B 13/02**

(30) Priorität: 18.12.85 DE 3544816

(43) Veröffentlichungstag der Anmeldung:
01.07.87 Patentblatt 87/27

(84) Benannte Vertragsstaaten:
AT BE CH FR GB IT LI NL SE

(71) Anmelder: Vereinigte Papierwerke AG
Schoppershofstrasse 80
D-8500 Nürnberg(DE)

(72) Erfinder: Männlein, Elisabeth, Dr.
Franzosenweg 24
D-85320 Erlangen(DE)

(72) Erfinder: Reinheimer, Horst, Dr.
Imkerweg 21
D-8501 Heroldsberg(DE)

(74) Vertreter: Pohl, Hans Ludwig
Hefnersplatz 3
D-8500 Nürnberg 11(DE)

(54) Höschenwindel mit verbesserter Dichtigkeit.

(57) Es wird eine zum einmaligen Gebrauch bestimmte Höschenwindel beschrieben, die eine flüssigkeitsdichte äussere Folie, eine Saugschicht und eine flüssigkeitsdurchlässige innere Abdeckung aufweist. Die äussere Folie un die innere Abdeckung sind breiter und länger als die Saugschicht und am überstehenden Rand miteinander verbunden. Zur Erhöhung des Flüssigkeitsaufnahmevermögens ist die äussere Folie (2) breiter also die innere Abdeckung (4) und die grössere Breite ist durch wenigstens eine in Längsrichtung verlaufende Falte (6) ausgeglichen. Diese Falte soll vorzugsweise eine Kellerfalte sein, welche an den beiden Enden (8;8') durch je eine Schweissnaht (9;9') stabilisiert ist.

Fig.1

EP 0 226 789 A2

Vereinigte Papierwerke
Schickedanz & Co.

0226789

Schoppershofstr. 80
8500 Nürnberg

12.12.85  -  W 47 D
HP/1

1

## Höschenwindel mit verbesserter Dichtigkeit

Die Erfindung betrifft eine zum einmaligen Gebrauch bestimmte Höschenwindel mit einer flüssigkeitsundurchlässigen äußeren Folie, einer Saugschicht und einer flüssigkeitsdurchlässigen inneren Abdeckung, wobei die äußere
Folie und die innere Abdeckung breiter und länger sind
als die Saugschicht und am überstehenden Rand miteinander
verbunden sind.

Derartige Höschenwindeln sind grundsätzlich bekannt
(DE-AS 14 35 891). Sie werden in verschiedenen Ausführungsformen hergestellt, wobei das Windelblatt im völlig
ausgebreiteten flachgelegten Zustand entweder die Form
eines Rechteckes aufweist oder sanduhrförmig zugeschnitten ist. Bei der zuerst genannten rechteckigen Grundrißform wird die Windel meist als Ganzes mit einer in Längsrichtung verlaufenden Kellerfalte ausgerüstet, wodurch
sie natürlich schmäler wird. Beim Anlegen der Windel
bleibt die Verschmälerung im Schrittbereich erhalten und
an den überstehenden Bereichen, die bei Gebrauch die Rükken- und Bauchpartie des Kindes bedecken, wird die Verschmälerung durch Auseinanderziehen der Kellerfalte wieder aufgehoben. Bei sanduhrförmig geschnittenen Windeln
ist eine Faltung zur Verschmälerung des Schrittbereiches
nicht erforderlich, da hier die nötige Verjüngung durch
die sanduhrförmigen Einschnitte bereits erreicht ist.

Vereinigte Papierwerke
Schickedanz & Co.

Schoppershofstr. 80
8500 Nürnberg

12.12.85 - W 47 D
HP/2

2
- 8 -

Es ist bekannt, daß die erwähnten beiden Windelformen
geeignet sind, erhebliche Flüssigkeits- und Feststoff-
Mengen aufzunehmen. Indessen besteht nach wie vor die
Gefahr, daß Verunreinigungen nach außen dringen, insbesondere dann, wenn diese stoßartig anfallen. Die Windel
ist dann manchmal nicht in der Lage, die plötzlich anfallenden Flüssigkeitsmengen schnell genug im Saugkörper zu
verteilen, so daß partiell eine Obersättigung des Saugkörpers eintritt, womit die Gefahr verbunden ist, daß
Flüssigkeit durch die Beinöffnungen nach außen gelangt.
Nach Lage der Dinge ist diese Gefahr bei sanduhrförmig
zugeschnittenen Windeln größer als bei den zuvor genannten Windeln mit Kellerfalte.

Der Erfindung liegt die Aufgabe zugrunde, Höschenwindeln
der beschriebenen Art (Rechteckform oder Sanduhrform)
derart weiterzuentwickeln, daß eine Windel mit erhöhter
Auslaufsicherheit entsteht.

Zur Lösung dieser Aufgabe wird eine Windel vorgeschlagen,
deren flüssigkeitsundurchlässige äußere Folie breiter ist
als die innere Abdeckung und bei der die größere Breite
der äußeren Folie durch wenigstens eine in Längsrichtung
verlaufende Falte ausgeglichen ist. Durch diese in Längsrichtung verlaufende Falte entsteht ein Raum, der zur
Aufnahme von plötzlich anfallenden Ausscheidungen und
auch von Saugstoff geeignet ist. Dadurch, daß die Falte

- 4 -

Vereinigte Papierwerke
Schickedanz & Co.

Schoppershofstr. 80
8500 Nürnberg

12.12.85 - W 47 D
HP/3

3
- 8 -

in Längsrichtung der Windel verläuft, wird zudem noch
begünstigt, daß die anfallenden Ausscheidungen möglichst
schnell dahin verteilt werden, wo sich der meiste Saugstoff befindet.

Grundsätzlich ist es möglich, beim vorgeschlagenen Windelkonzept mit einer einzigen in Längsrichtung verlaufenden Falte auszukommen. Vorteilhafter ist es jedoch, mehrere Falten vorzusehen, welche dann parallel nebeneinander angeordnet sein können und beispielsweise eine Kellerfalte bilden. Diese Kellerfalte erfaßt aber nur die
äußere flüssigkeitsundurchlässige Folie und nicht etwa,
wie bei bekannten Windeln, das gesamte aus Folie, Saugschicht und flüssigkeitsdurchlässiger Abdeckung bestehende Saugpaket.

Desweiteren wird vorgeschlagen, die Falten, beispielsweise die erwähnte Kellerfalte, an ihren beiden Enden durch
je eine Schweißnaht zu stabilisieren. Durch diese Stabilisierung wird sichergestellt, daß die Falten in der gewünschten Lage liegenbleiben, was für das ordentliche
Aussehen der Windel, insbesondere im verpackten Zustand
aber auch im Gebrauchszustand, wichtig ist. Zusätzlich
können, falls erwünscht, noch Klebepunkte im Bereich der
Faltungen vorgesehen sein, wobei diese Klebepunkte unter
Verwendung eines Klebstoffes hergestellt werden, der zwar
die Faltung in seiner Lage stabilisiert, der aber ande-

Vereinigte Papierwerke        Schoppershofstr. 80
Schickedanz & Co.            8500 Nürnberg

12.12.85  -  W 47 D
HP/4

4
-ᴢᴠ-

rerseits leicht aufreißt, wenn bei Gebrauch der Faltung
der durch die Faltung zur Verfügung gestellte Raum ausgenützt werden muß.

Die Erfindung wird nachfolgend anhand der beigefügten
Zeichnung näher erläutert.

Es stellen dar:

Fig. 1 eine Draufsicht einer Ausführungsform der Windel;
Fig. 2 einen Querschnitt entlang der Linie II-II der
        Fig. 1.

Die in der Zeichnung als Beispiel dargestellte Windel ist
als Ganzes mit 1 bezeichnet. Sie weist eine flüssigkeitsundurchlässige äußere Folie 2, eine Saugschicht 3 sowie
eine flüssigkeitsdurchlässige innere Abdeckung 4 auf. Als
flüssigkeitsundurchlässige äußere Folie kann beispielsweise eine Polyethylenfolie von 25 - 100 µ/m Stärke verwendet werden. Diese Folie kann oberflächlich aufgerauht
sein, um ihr einen angenehmeren textilähnlicheren Griff
und ein textilähnlicheres Aussehen zu verleihen. Als
Saugschicht wird meistens ein Vlies aus Zellstoff-Flocken
verwendet, welches aber gegebenenfalls auch noch andere
Saugstoffe, beispielsweise sogenannte Quellstoffe enthalten kann. Anstelle eines Flockenvlieses könnten auch mehrere Lagen von weichem, saugfähigem Tissuepapier verwen-

-ᴢᴢ-

Vereinigte Papierwerke     Schoppershofstr. 80  0226789

Schickedanz & Co.          8500 Nürnberg

                           12.12.85  - W 47 D
                           HP/5

- 2 -

det werden. Als flüssigkeitsdurchlässige innere Abdeckung
kommen Vliesstoffe in Betracht, also Vliese aus textilen
Fasern, die an ihren Kreuzungsstellen durch Klebstoffe,
Verschweißen, Vernadeln oder ähnliche Art miteinander
verbunden sind.

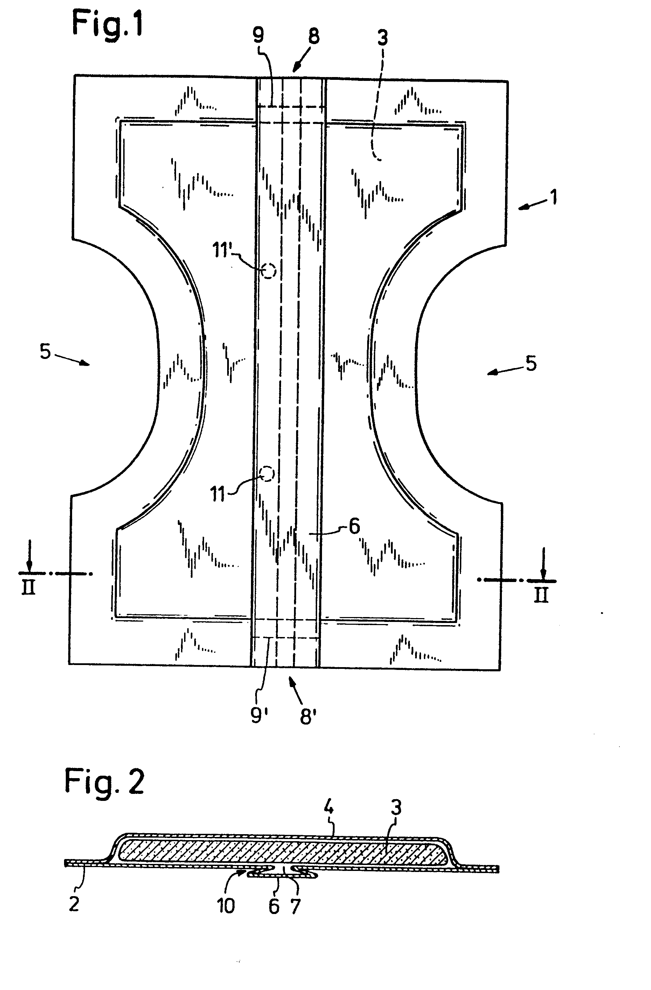

Die Windel als Ganzes kann in der Draufsicht rechteckförmig oder auch, wie in Fig. 1 als Beispiel dargestellt,
sanduhrförmig geschnitten sein. Der sanduhrförmige
Schnitt hat einerseits den Vorteil, daß im Bereich der
Beinöffnungen 5 die erforderliche Schrittverengung sogleich vorliegt, und daß andererseits Material, insbesondere Saugstoff bei der Herstellung der Windel gespart
wird. Andererseits besteht aber gerade bei derartigem
sanduhrförmigen Schnitt der Nachteil, daß im Schrittbereich das Flüssigkeitsaufnahmevermögen der Windel besonders gering ist, obgleich doch an dieser Stelle die aufzunehmende Flüssigkeit anfällt.

Erfindungsgemäß ist die Windel dadurch gekennzeichnet,
daß die flüssigkeitsundurchlässige äußere Folie 2 breiter
ist als die innere Abdeckung 4 und die größere Breite
durch wenigstens eine in Längsrichtung verlaufende Falte 6 ausgeglichen ist. Dadurch entsteht im Bereich der
Falte zusätzlicher Raum 7, der zur vorübergehenden Aufnahme plötzlich anfallender Ausscheidungen geeignet ist
und in den sich auch die Saugschicht 3 bei Beladung mit
Flüssigkeit oder dergl. ausdehnen kann.

0226789

Vereinigte Papierwerke    Schoppershofstr. 80
Schickedanz & Co.        8500 Nürnberg

12.12.85  -  W 47 D
HP/6

- 7 -

Im dargestellten Ausführungsbeispiel ist die Falte 6 eine
Kellerfalte. Eine derartige Kellerfalte ist zwar vorteilhaft, jedoch im vorliegenden Zusammenhang nicht unerläßlich. An ihre Stelle kann auch eine einfache Z-Falte treten oder gegebenenfalls auch mehrere Z-Faltungen, welche
nebeneinander angeordnet werden.

Desweiteren wird vorgeschlagen, die Falten 6 an ihren
beiden Enden 8 und 8' durch je eine Schweißnaht 9 und 9'
zu stabilisieren. Zusätzlich können zur weiteren Stabilisation noch Klebepunkte angeordnet werden, beispielsweise
in den Kehlen 10 der Kellerfalte und etwa in den Positionen 11 und 11' der Fig. 1.

Um die Wirkung der erfindungsgemäß vorgeschlagenen Kellerfalte in der äußeren Abdeckung zu demonstrieren, wurden Vorlagen hergestellt, die jeweils in ihrem grundsätzlichen Aufbau der Fig. 1 entsprachen. Die eine Hälfte
dieser Versuchsvorlagen wurde in herkömmlicher Weise ohne
Kellerfalte in der äußeren Abdeckung und die andere Hälfte mit Kellerfalte in der äußeren Abdeckung hergestellt.
Die Versuchsvorlagen wurden einer Meßpuppe angelegt und
mittels einer Netzhose befestigt. Die Vorlagen wurden
dann jeweils mit wässriger Harnersatzlösung beaufschlagt,
wobei die Fließgeschwindigkeit ca. 20 ml pro Minute betrug. Es wurde diejenige Menge an Flüssigkeit gemessen,
die die Vorlage ohne überzulaufen aufnehmen konnte.

- 8 -

0226789

Vereinigte Papierwerke          Schoppershofstr. 80
Schickedanz & Co.               8500 Nürnberg

                                12.12.85  -  W 47 D
                                HP/7

Ergebnis:

Vorlage ohne Kellerfalte 110 ml (Mittelwert)
Vorlage mit Kellerfalte  350 ml (Mittelwert).

Die Versuchsergebnisse zeigen, daß die Muster mit Kellerfalte mehr als das Doppelte an Flüssigkeit aufnehmen
konnten, wobei der besondere Vorteil darin bestand, daß
auch rasch anfallende Flüssigkeitsmengen sicher gehalten
werden bis sie vom Saugkissen aufgenommen werden können.

Vereinigte Papierwerke
Schickedanz & Co.

Schoppershofstr. 80
8500 Nürnberg

12.12.85  -  W 47 D

8

## <u>Bezugszeichenliste</u>

| | | |
|---|---|---|
| 1 | = | Windel |
| 2 | = | äussere Folie |
| 3 | = | Saugschicht |
| 4 | = | innere Abdeckung |
| 5 | = | Beinöffnung |
| 6 | = | Falte |
| 7 | = | Raum |
| 8; 8' | = | Enden der Falte (6) |
| 9; 9' | = | Schweissnaht |
| 10 | = | Kehle |
| 11; 11 | = | Positionen v. Klebepunkten |

Vereinigte Papierwerke          Schoppershofstr. 80
Schickedanz & Co.               8500 Nürnberg

                                12.12.85  - W 47 D
                                HP/8


Patentansprüche


1. Zum einmaligen Gebrauch bestimmte Höschenwindel mit
   einer flüssigkeitsundurchlässigen äußeren Folie, einer
   Saugschicht und einer flüssigkeitsdurchlässigen inneren Abdeckung,
   wobei die äußere Folie und die innere Abdeckung breiter und länger sind als die Saugschicht und am überstehenden Rand miteinander verbunden sind,
   dadurch gekennzeichnet,
   daß die flüssigkeitsundurchlässige äußere Folie (2)
   breiter ist als die innere Abdeckung (4) und die größere Breite durch wenigstens eine in Längsrichtung
   verlaufende Falte (6) ausgeglichen ist.


2. Höschenwindel nach Anspruch 1,
   dadurch gekennzeichnet,
   daß die Falte (6) eine Kellerfalte ist.


3. Höschenwindel nach Anspruch 1 oder 2,
   dadurch gekennzeichnet,
   daß die Falten (6) an ihren beiden Enden (8; 8') durch
   je eine Schweißnaht (9; 9') stabilisiert sind.

0226789

Fig.1

Fig. 2

W 47 D